# EUROPEAN PATENT SPECIFICATION

(11) **EP 3 200 910 B1**
(45) Date of publication and mention of the grant of the patent: **07.06.2023**
(21) Application number: 15760075.0
(22) Date of filing: 02.09.2015
(51) Int. Cl.: B01J 19/00, C07C 253/18, C07C 253/34, C07C 255/08, B01D 1/00, B01D 1/26, B01D 3/14

(54) **EVAPORATION SYSTEM FOR A PROCESS STREAM**
VERDAMPFUNGSSYSTEM FÜR EINEM PROZESSSTROM
SYSTÈME D'ÉVAPORATION POUR FLUX DE TRAITEMENT

(30) Priority: 29.09.2014 CN 201410511314
(43) Date of publication of application: 09.08.2017
(60) Divisional application: 17202343.4
(73) Proprietor: Ineos Europe AG, 1180 Rolle (Vaud) (CH)
(72) Inventor: MCDONEL, Timothy Robert, Elburn, Illinois 60119 (US); COUCH, Jay Robert, Naperville, Illinois 60564 (US); WAGNER, David Rudolph, Naperville, Illinois 60564 (US); WACHTENDORF, Paul Trigg, Victoria, Texas 77904 (US)
(74) Representative: King, Alex
(86) International application number: PCT/US2015/048029
(87) International publication number: WO 2016/053559

(56) References cited:
- EP-A1- 3 300 784
- GB-A- 1 492 128
- US-A- 3 636 068
- US-A- 3 734 943
- US-A- 4 166 008
- US-A- 4 334 965
- US-A- 4 377 444
- US-A1- 2004 267 075

## Description

### FIELD OF THE INVENTION

The invention is defined by the appended claims. Parts of the disclosed process not encompassed by the wording of the claims are considered useful for understanding the invention. A process for removing heavy organic impurities from a process stream is provided. More specifically, the process includes providing a process stream and separating water from heavy organics from the process stream in an evaporator system. The evaporator system is effective for providing an aqueous condensate and liquid residue.

### BACKGROUND

Various processes and systems for the manufacture of acrylonitrile and methacrylonitrile are known; see for example, U.S. Patent No. 6,107,509. Typically, recovery and purification of acrylonitrile/methacrylonitrile produced by the direct reaction of a hydrocarbon selected from the group consisting of propane, propylene or isobutylene, ammonia and oxygen in the presence of a catalyst has been accomplished by transporting the reactor effluent containing acrylonitrile/methacrylonitrile to a first column (quench) where the reactor effluent is cooled with a first aqueous stream, transporting the cooled effluent containing acrylonitrile/methacrylonitrile into a second column (absorber) where the cooled effluent is contacted with a second aqueous stream to absorb the acrylonitrile/methacrylonitrile into the second aqueous stream, transporting the second aqueous stream containing the acrylonitrile/methacrylonitrile from the second column to a first distillation column (recovery column) for separation of the crude acrylonitrile/methacrylonitrile from the second aqueous stream, and transporting the separated crude acrylonitrile/methacrylonitrile to a second distillation column (heads column) to remove at least some impurities from the crude acrylonitrile/ methacrylonitrile, and transporting the partially purified acrylonitrile/methacrylonitrile to a third distillation column (product column) to obtain product acrylonitrile/methacrylonitrile. U.S. Pat. Nos. 4,234,510; 3,885,928; 3,352,764; 3,198,750 and 3,044,966 are illustrative of typical recovery and purification processes for acrylonitrile and methacrylonitrile.

A process for recovery of olefinic nitriles is described in U.S. Pat. No. 4,334,965. As described in U.S. Pat. No. 4,334,965, a multi-stage evaporator is used to remove water from the extracted distillation or stripper tower bottoms recycled as quench liquid to the quench tower of an acrylonitrile purification and recovery system. This process results in a significant decrease in the amount of waste quench tower bottoms produced by the system. Use of a multi-effect evaporator represents a significant energy savings as compared with other techniques for decreasing the water content of the recycle stream. U.S. Pat. No. 4,334,965 discloses that by means of a multi-effect evaporator, 50% or more of the liquid in the recycled stream can be removed therefrom leaving a concentrated recycle stream to serve as the quench liquid in the same way as shown in U.S. Pat. No. 4,166,008. U.S. Pat. No. 4,334,965 discloses that because multi-effect evaporators are so energy efficient, however, the overall energy costs of this technique are much lower than the technique described in U.S. Pat. No. 4,166,008.

While the manufacture of acrylonitrile/methacrylonitrile has been commercially practiced for years there are still areas in which improvement would have a substantial benefit. One of those areas of improvement would be more efficient evaporator operation for recovery column bottoms.

### SUMMARY

Accordingly, an aspect of the disclosure is to provide a safe, effective and cost effective process and apparatus that overcomes or reduces the disadvantages of conventional processes.

A process for removing heavy organic impurities from a process stream includes providing a process stream having water and about 0.5 to about 1.5 weight percent heavy organic impurities. The process includes separating water from the heavy organic impurities in an evaporator system having one or more evaporation stages to provide an aqueous condensate and a liquid residue. The aqueous condensate has about 0.1 weight percent or less heavy organic impurities and the liquid residue has about 3 to about 10 weight percent heavy organic impurities.

A process for providing a liquid residue to an ammoxidation process stream, includes providing a process stream that includes water and heavy organic impurities; separating water from the heavy organic impurities in an evaporator system having one or more evaporation stages to provide an aqueous condensate and a liquid residue; and contacting the liquid residue with a reactor effluent in a quench column to provide ammonium sulfate. An amount of ammonium sulfate and an amount of polymer are defined by a formula y = -M1x + C1 where y is a weight % of ammonium sulfate, x is a weight % polymer, M1 is 4.6 or less and C1 is 45 or less.

An evaporator system includes one or more evaporation stages, wherein a first evaporation stage is configured to receive a process stream from a distillation column, the one or more evaporation stages configured to provide an aqueous condensate and a liquid residue; and a quench column and/or light organic stripper configured to receive aqueous condensate.

An evaporation process includes conveying a process stream from a distillation column to an evaporator system, the evaporator system including one or more evaporation stages, wherein a first evaporation stage is configured to receive the process stream from the distillation column, providing an aqueous condensate and a liquid residue from the one or more evaporation stages; and conveying aqueous condensate to a quench column and/or light organic stripper.

The above and other aspects, features and advantages of the present disclosure will be apparent from the following detailed description of the illustrated embodiments thereof which are to be read in connection with the accompanying drawings.

### BRIEF DESCRIPTION OF THE DRAWINGS

A more complete understanding of the exemplary embodiments of the present disclosure and the advantages thereof may be acquired by referring to the following description in consideration of the accompanying drawing in which like reference numbers indicate like features and wherein:
FIG. 1 is a schematic flow diagram of a process for the manufacture of acrylonitrile product (not part of the invention).
FIG. 2 is a schematic flow diagram of an alternative process for the manufacture of acrylonitrile product.

### DETAILED DESCRIPTION

### Ammoxidation Process

In one aspect, a process stream is provided from an ammoxidation reaction process. An example of one such process is described in U.S. Pat. No. 4,334,965.

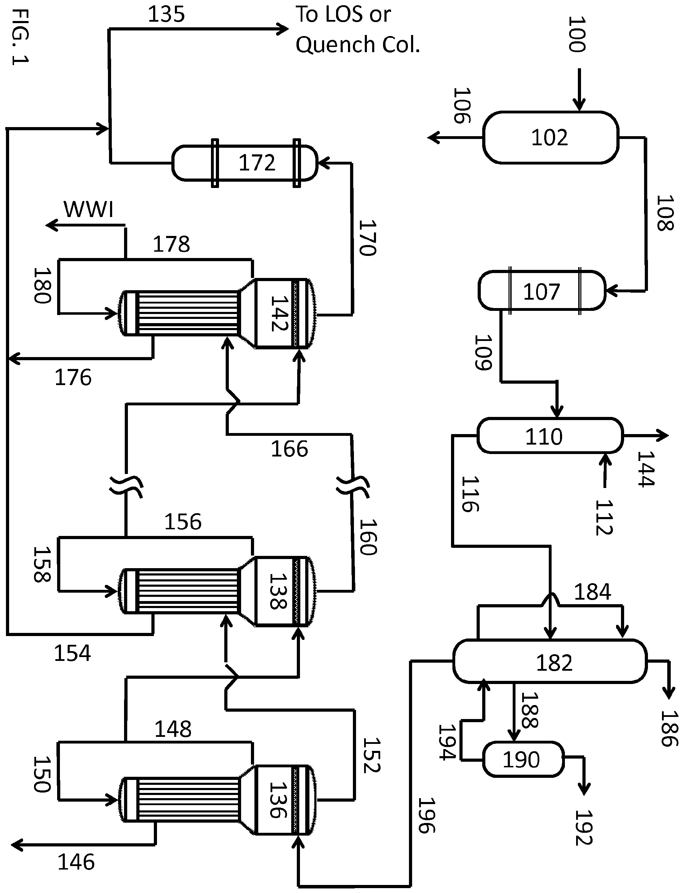

Figure 1 is an overall schematic representation of various aspects (not part of the invention). Referring to FIG. 1, the reactor effluent gas in conduit 100 comprising acrylonitrile, HCN, acetonitrile, water vapor and impurities may be first passed to a quench column 102. The gas may be contacted with quench liquid in quench column 102. A bottoms stream containing water and impurities may be removed through conduit 106 and sent to waste treatment.

The cooled reactor effluent gases may leave the quench system through line 108 and pass to quench aftercooler 107. The quench aftercooler 107 is effective for cooling the quench effluent to below about 50 °C. Cooled quench effluent is feed through line 109 to absorber 110. Wash water may enter absorber 110 at the top through line 112. Non-condensable gases may be removed from the absorber through line 114. An aqueous solution containing water, acrylonitrile, acetonitrile and impurities may be removed as a bottoms stream through line 116 and passed to extractive distillation column 182.

Solvent water may be introduced to the top of extractive distillation column 182 through line 184 to perform extractive distillation. Acrylonitrile and HCN may be removed as an overhead vapor through line 186 and sent to further purification (not shown). A stream containing acetonitrile and water may be removed through line 188 and passed to stripper 190. Heat may be added to stripper 190 to remove acetonitrile as an overhead vapor through line 192. The bottoms stream containing water, heavy organics and other impurities may be removed from extractive distillation column 182 through line 196. A liquid stream containing mostly water may be removed from the lower half of stripper 190 through line 194 and used as solvent water to extractive distillation column 182.

### Evaporator System

In accordance with one aspect, stripper column bottoms in line 196, which may also be referred to as a process stream, may be subjected to evaporation in an evaporator system. In this aspect, the extractive distillation column bottoms in line 196 that may enter heat exchanger 136 comprises water, polymer, ammonia, and acrylonitrile. As used herein, "heavy organic impurities" refers to polymer. As used herein polymer refers to a mixture of heavy organic material and a slight amount of light organics. Heavy organic material may include a mixture of different high boiling organic compounds having a high degree of nitrile substitution and also containing some oxygenated hydrocarbon groups. In this aspect, the process stream includes about 0.5 to about 1.5 weight percent heavy organic impurities, and in another aspect, about 0.75 to about 1.25 weight percent.

In this aspect, the evaporator system may include one or more evaporation stages effective for providing an aqueous condensate and liquid residue. For example, the evaporator system may include 1 to about 6 evaporation stages, in another aspect, 2 to about 6 evaporation stages, in another aspect, 2 to about 5 evaporation stages, in another aspect, 2 to about 4 evaporation stages, and in another aspect, 2 to about 3 evaporation stages.

In one aspect illustrated in Figure 1, an evaporator system includes shell and tube heat exchangers 136, 138 and 142 arranged in series. As used herein, an "evaporation stage" refers to a single heat exchanger. In each heat exchanger, liquid in the tube side of the exchanger is partially evaporated producing a vaporous effluent and a liquid effluent. The liquid effluent is fed to the tube side of the next heat exchanger in the series while the vaporous effluent is fed to the shell side of the same heat exchanger causing additional partial evaporation of the liquid. This technique is continued for as many stages as is necessary to remove the desired amount of water from the stripper bottoms. In each stage, condensate produced when the heat-supplying vapor is condensed through heat exchange is recovered and either recycled for reuse or subjected to chemical or biological purification.

Bottoms stream containing water, heavy organics and other impurities may be removed from extractive distillation column 182 through line 196 and passed into the tube side of first heat exchanger 136, while low pressure steam is passed through the shell side of this heat exchanger. In one aspect, a flow through the tube side of the heat exchanger is about 1 to about 3 meters/second, and in another aspect, about 1.5 to about 2.5 meters/second. Heat exchange therein causes the lower pressure steam to condense and the extractive distillation column bottoms to partially evaporate. Condensate may be removed from first heat exchanger 136 for reuse via line 146.

Heating of the extractive distillation column bottoms in first heat exchanger 136 causes partial separation thereof into vapor and liquid phases. In aspects where more than one heat exchanger is used, the liquid phase is withdrawn via line 148 and transferred to the tube side of second heat exchanger 138, a portion of the withdrawn liquid being recycled via line 150 to the bottom of the tube side of first heat exchanger 136. Vapor produced in first heat exchanger 136 is withdrawn and transferred via line 152 to the shell side of second heat exchanger 138. Heat exchange in heat exchanger 138 causes condensation of the vapor on the shell side and partial evaporation of the liquid in the tube side, thereby producing liquid in vapor phases in second heat exchanger 138. Condensate produced on the shell side of second heat exchanger 138 is discharged via line 154. This condensate has a relatively low concentration of heavy organics such as polymer and the like.

In aspects, where more than two heat exchangers are used, the liquid phase remaining in the tube side of second heat exchanger 138 is transferred via line 156 to the tube side of third heat exchanger 142, a portion of the liquid being recycled via line 158 to the tube side of second heat exchanger 138. Vapor produced in the tube side of second heat exchanger 138 is transferred via line 160 to the shell side of third heat exchanger 142. Again, heat exchange in third heat exchanger 142 causes condensation of the vapor on the shell side to form a condensate which is withdrawn via line 176 and conveyed in the same way as the condensate from second heat exchanger 138.

The vapor produced in the tube side of third heat exchanger 142 is withdrawn via line 170, condensed in condenser 172, and recovered in a utility water vessel and /or combined with condensate from lines 146, 154, 162, and/or 176. The condensate from the shell side of heat exchanger 142 may also be transferred to the utility water vessel via line 176, for example, as combined and provided at 135. Liquid recovered from the tube side of third heat exchanger 142 may be withdrawn via line 178 and recycled via line 180 to the tube side of the third heat exchanger. Aqueous condensates may be of such high purity that they may be used as conventional clean water such as, for example, in the flushing of various process equipment, as a return to the quench column (for example a first stage of a quench column), and/or to the light organic stripper. In this aspect, aqueous condensate has about 0.1 weight percent or less heavy organic impurities, in another aspect, about 0.075 heavy organic impurities, in another aspect, about 0.05 heavy organic impurities, and in another aspect, about 0.025 heavy organic impurities.

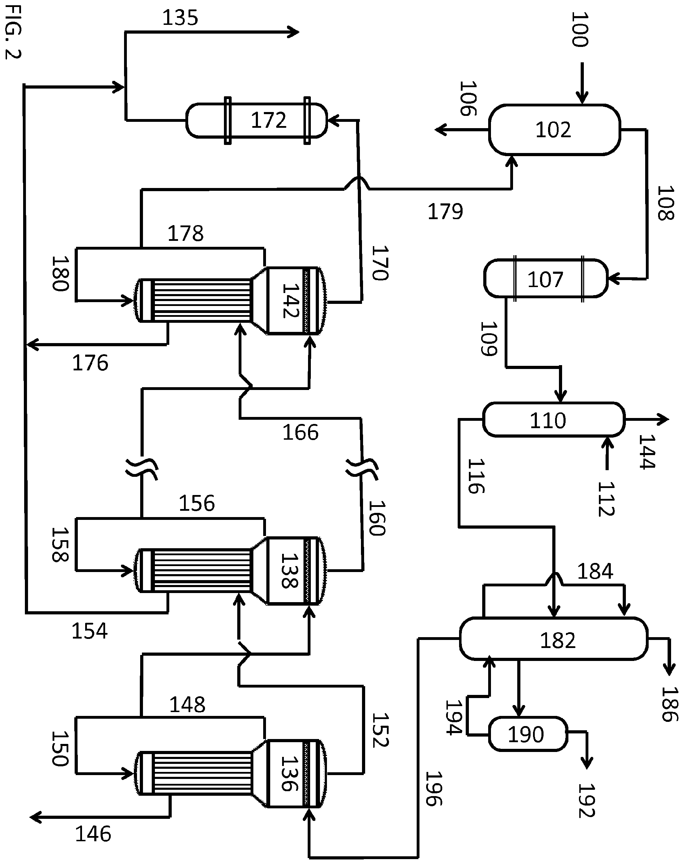

In another aspect, the liquid residue has about 3 to about 10 weight percent heavy organic impurities, in another aspect, about 4 to about 8 weight percent heavy organic impurities, and in another aspect, about 5 to about 7 weight percent heavy organic impurities. As shown in Figure 1, liquid residue may be sent to a waste water incinerator (WWI) (not part of the invention). As shown in Figure 2, the liquid residue may be sent via line 179 to a lower portion of a quench tower 102.

In one aspect, the aqueous condensates produced in the second and third heat exchangers contain extremely small amounts of heavy organics. Thus, they can be directly processed by conventional biological or chemical treatment to produce environmentally acceptable water. Furthermore, the condensate produced in the fourth heat exchanger as well as the condensate produced by the condenser are pure enough to be used for various process purposes such as wash water without further treatment. The condensate produced by the first heat exchanger, since it does not contact any other process stream, is highly pure.

### Evaporation Percentage

In one aspect, higher evaporation may be beneficial because the evaporator condensate may be reused or disposed of while the liquid residue from the last stage of the multi-stage evaporator may be incinerated and/or otherwise disposed of.

In an aspect, the evaporation percentage of the bottoms of extractive distillation column may be greater than about 55% to about 85%. In an aspect, the evaporation percentage of the bottoms of extractive distillation column may be greater than about 60%. In an aspect, the evaporation percentage of the bottoms of extractive distillation column may be greater than about 60% to about 85%. In an aspect, the evaporation percentage of the bottoms of extractive distillation column may be in the range of about 73% to about 75%.

By running a four (4) stage evaporation process with about a 55 to about 60%, in one aspect, about a 57% vaporization percentage, the percent of liquid polymer in the evaporator bottoms coming out of the fourth and last heat exchanger 142 may be about 2.2% by weight.

By running a four (4) stage evaporation process with about a 60-65%, in one aspect, about 63% vaporization percentage, the percent of liquid polymer in the evaporator bottoms coming out of the fourth and last heat exchanger 142 may be about 3% by weight.

By running a four (4) stage evaporation process with about a 80-85%, in one aspect about 83% vaporization percentage, the percent of liquid polymer in the evaporator bottoms coming out of the fourth and last heat exchanger 142 may be about 6% by weight.

By running a four (4) stage evaporation process with about a 73-75%, in one aspect about 74% vaporization percentage, the percent of liquid polymer in the evaporator bottoms coming out of the fourth and last heat exchanger 142 may be about 5.5% by weight.

In an aspect, each evaporative stage provides an evaporation rate of about 15 to about 25%.

In an aspect, the percentage of evaporator condensate divided by feed percentage may be the evaporation percentage of the bottoms of extractive distillation column 182. In one aspect, the evaporation percentage is about 55 to about 60%, and in another aspect, about 57%. The amount of polymer in the liquid residue is about 2.2% by weight. In an aspect, the ratio of the percent evaporation percentage to percent by weight of polymer is about 55-60:2.2.

In another aspect, the amount of polymer in the liquid residue is about 3% by weight. In an aspect, the ratio of the percent evaporation percentage to percent by weight of polymer is about 60-65:3.

In an aspect, the evaporation percent is about 82 to about 83%, and the amount of polymer in the liquid residue is about 6.0% by weight. The condensate may be fed as feed to a light organic stripper LOS (not shown) via line 135 for further processing, or sent to quench column 102 as quench liquid. In an aspect, the ratio of the percent evaporation percent to percent by weight of polymer is about 82-83:6.

In an aspect, the evaporation percent is about 73 to about 75% and the polymer in the liquid residue is about 5.5% by weight. The condensate may be feed as feed to a light organic stripper LOS (not shown) via line 135 for further processing, or sent to quench column 102 as quench liquid. In an aspect, the ratio of the percent evaporation percent to percent by weight of polymer is about 73-75:5.5.

In an aspect, it was found that when the evaporation percent is about 74%, substantially less fouling was experienced than at an evaporation percent of about 83%, while at the same time achieving a relatively high amount of polymer by weight in the liquid residue, i.e., 5.5% by weight, versus 6.0% by weight at the evaporation percent of about 83%. This is a surprising result, as it would have been expected that the amount of fouling would be linearly related to the percent by weight of polymer in the liquid stream.

It has been found that when the evaporation percent is greater than about 83%, there may be too much fouling (mostly on the tube side) in the fourth stage evaporator or heat exchanger 142. It has been found that an evaporation percent of about 73-75% significantly reduces the amount of fouling, while at the same time, providing a relatively high amount of polymer by weight in the liquid residue.

Those skilled in the art will recognize that in accordance with the present disclosure, many modifications can be made. For example, any number of stages can be employed in the evaporator system. Furthermore, although low pressure steam is shown in the above description as supplying the heat necessary for all the evaporations, any heat source can be employed. In a typical acrylonitrile purifications and recovery plant, however, low pressure steam, that is saturated steam having a pressure of up to 6.894 barg (100 psig), normally about 1.379 to 4.137 barg (20 to 60 psig), is readily available and is preferably used. Also, the amount of water in the stripper column removed by the multiple effect evaporator can be varied depending primarily upon economics. Finally, it should also be appreciated that the multiple effect evaporator of this disclosure need not be restricted to use on stripper column bottoms as shown in the above description, but can be employed to concentrate any other process stream which is recycled for use as the quench liquid. For example, a multi-effect evaporator can be used to process the extractive distillation tower bottoms recycled in line 156 of FIG. 2 of U.S. Pat. No. 4,166,008.

### Quench Column Operation

In one aspect, a process for operating a quench column includes conveying a reactor effluent to a quench column and contacting the reactor effluent with water containing polymer in an effluent extraction zone to provide an extracted effluent stream. The process further includes contacting the extracted effluent stream with sulfuric acid in an acid contact zone and removing a first stream to provide a first quench column stream having about 10 weight percent or less polymer. In one aspect, the process includes providing at least a portion of the water from an evaporator system. As described herein, the water may be an aqueous condensate (not part of the invention) and/or a liquid residue.

Formula y = -M1x + C1 where y is a weight % of ammonium sulfate, x is a weight % polymer, M1 is 4.6 or less and C1 is 45 or less defines an amount of ammonium sulfate and an amount of polymer in the quench column bottoms stream. In an related aspect, M1 is 1.5 or less and C1 is 30 or less. In one aspect, the process provides a quench column bottoms streams having about 10 to about 25 weight % ammonium sulfate and less than about 5 weight % polymer, in another aspect, about 15 to about 21 weight % ammonium sulfate and less than about 5 weight % polymer. The quench column bottoms stream has a pH of about 4.5 to about 6.0.

In another aspect, the process includes removing a second stream to provide a second quench column stream having more than about 10 weight percent polymer and less than about 5 weight percent ammonium sulfate.

In another aspect, at least a portion of the second quench column stream is recycled to the effluent extraction zone. The extracted effluent stream is countercurrent to the sulfuric acid. In one aspect, a first quench column stream is removed above the effluent extraction zone. Adiabatic cooling may occur in the effluent extraction zone.

In an aspect, a process may comprise controlling the amount of makeup water conveyed to the quench column and/or controlling the amount of sulfuric acid conveyed to the quench column to obtain a concentration of ammonium sulfate in a quench column bottoms stream of about 10 to about 25% by weight. In an aspect, the process may comprise detecting the pH of the liquid bottoms of the quench column bottoms and controlling the flow of sulfuric acid to the quench column based on the detected pH of the liquid bottoms to obtain a quench column bottoms stream having a pH of about 4.5 - 6.0. In an aspect, the process may comprise determining the concentration of ammonium sulfate in the quench column bottoms stream based on the flow rate of sulfuric acid to the quench column and the flow rate of the quench column bottoms stream from or out of the quench column. In an aspect, the process may comprise adjusting the flow rate of sulfuric acid and/or makeup water conveyed to the quench column based upon the concentration of ammonium sulfate determined in the determining step to maintain the concentration of ammonium sulfate in the range of about 10 to about 25% by weight. By increasing the concentration of ammonium sulfate in the quench column bottoms stream to about 10 to about 25% by weight from the 5-10% by weight provided in a conventional process, less water needs to be removed from the quench columns bottom stream to obtain even higher concentrations of ammonium sulfate. It has been found that by increasing the concentration of ammonium sulfate in the quench column bottoms stream to about 10 to about 25% by weight, a sulfate condenser may be used to efficiently condense the quench column bottoms stream to about 35-40% by weight.

While in the foregoing specification this disclosure has been described in relation to certain preferred embodiments thereof, and many details have been set forth for purpose of illustration, it will be apparent to those skilled in the art that the disclosure is susceptible to additional embodiments and that certain of the details described herein can be varied considerably . For example, the dimensions, number, size and shape of the various components may be altered to fit specific applications. Accordingly, the specific embodiments illustrated and described herein are for illustrative purposes only.

## Claims

1. A process for providing a liquid residue to an ammoxidation process stream, the process comprising:
passing a reactor effluent gas comprising acrylonitrile, HCN, acetonitrile, water vapor and impurities to a quench column (102),
contacting the reactor effluent gas with quench liquid in the quench column (102), to provide:
(a) a quench column bottoms stream containing water and impurities which is removed through a conduit (106) and sent to waste treatment, and
(b) a stream of cooled reactor effluent gases;
passing the cooled reactor effluent gases to a quench aftercooler (107),
feeding the cooled quench effluent from the quench aftercooler (107) to an absorber (110) and feeding wash water which enters at the top of the absorber, and removing from the absorber (110):
(a) non-condensable gases, and
(b) an aqueous solution containing water, acrylonitrile, acetonitrile and impurities which is removed as a bottoms stream;
passing the bottoms stream to an extractive distillation column (182), and introducing solvent water to the top of extractive distillation column (182) to perform extractive distillation, and removing from the extractive distillation column (182):
(a) acrylonitrile and HCN as an overhead vapor which is sent to further purification,
(b) a stream containing acetonitrile and water, and
(c) a bottoms stream containing water, heavy organics and other impurities;
passing the stream containing acetonitrile and water from the extractive distillation column (182) to a stripper (190), to which heat is added, and removing from the stripper (190):
(i) acetonitrile as an overhead vapor, and
(ii) a liquid stream containing mostly water from the lower half of stripper (190);
providing the bottoms stream containing water, heavy organics and other impurities from extractive distillation column (182) as a process stream (196) to an evaporator system having one or more evaporation stages,
separating water from the heavy organic impurities in the evaporator system having one or more evaporation stages to provide an aqueous condensate and a liquid residue, and
contacting the liquid residue with the reactor effluent in the quench column (102) to provide a quench columns bottom stream comprising ammonium sulfate and polymer;
wherein the amount of ammonium sulfate and the amount of polymer in the quench column bottoms stream are defined by a formula y = -M1x + C1 where y is a weight % of ammonium sulfate, x is a weight % polymer, M1 is 4.6 and C1 is 45 or less.

2. The process of claim 1 wherein the aqueous condensate has 0.1 weight percent or less heavy organic impurities and the liquid residue has 3 to 10 wt% heavy organic impurities.

3. The process of claim 1 wherein the process stream includes 0.5 to 1.5 weight percent heavy organic impurities, and preferably wherein the process stream includes 0.75 to 1.25 weight percent heavy organic impurities.

4. The process of claim 1 wherein the heavy organic impurities includes polymeric materials produced in an ammoxidation reaction process.

5. The process of claim 1 wherein the evaporator system includes 1 to 6 evaporation stages, preferably wherein the evaporator system includes 2 to 6 evaporation stages, such as wherein the evaporator system includes 2 to 5 evaporation stages, more preferably wherein the evaporator system includes 2 to 4 evaporation stages, and most preferably wherein the evaporator system includes 2 to 3 evaporation stages.

6. The process of claim 4 wherein the ammoxidation reaction process is an acrylonitrile process.

7. The process of claim 1 wherein the aqueous condensate has 0.075 weight percent or less heavy organic impurities, preferably wherein the aqueous condensate has 0.05 weight percent or less heavy organic impurities, and most preferably wherein the aqueous condensate has 0.025 weight percent or less heavy organic impurities.

8. The process of claim 1 wherein at least a portion of the aqueous condensate is conveyed to a quench column and/or a light organic stripper, and preferably wherein at least a portion of the aqueous condensate is conveyed to a first stage of a quench column.

9. The process of claim 1 wherein the evaporator system is effective for providing an overall evaporation percentage greater than 55% to 85%, preferably wherein the evaporator system is effective for providing an overall evaporation percentage greater than 60% to 85%, and most preferably wherein the evaporator system is effective for providing an overall evaporation percentage greater than 73% to 75%.

10. The process of claim 1 wherein the liquid residue has 4 to 8 weight percent heavy organic impurities, and preferably wherein the liquid residue has 5 to 7 weight percent heavy organic impurities.

11. The process of claim 1 wherein the evaporator system includes at least one shell and tube heat exchanger, and preferably wherein a flow through a tube side of the heat exchanger is 1 to 3 meters/second.

12. The process of claim 1 wherein each evaporative stage provides an evaporation rate of 15 to 25%.

13. The process of claim 1 wherein sulfuric acid is conveyed to the quench column to provide a quench column bottoms streams having 10 to 25 weight % ammonium sulfate and less than 5 weight % polymer, and preferably wherein sulfuric acid is conveyed to the quench column to provide a quench column bottoms streams having 15 to 21 weight % ammonium sulfate and less than 5 weight % polymer.

14. The process of claim 1 wherein the quench column bottoms stream has a pH of 4.5 to 6.0, and preferably wherein the pH is controlled with addition of acid.

15. The process of claim 1 wherein the waste treatment of the quench column bottoms stream involves removal of water to increase the concentration of ammonium sulfate therein.

## Patentansprüche

1. Verfahren zum Bereitstellen eines flüssigen Rückstands für einen Ammoxidationsprozessstrom, wobei das Verfahren Folgendes umfasst:
Leiten eines Reaktorabgases, das Acrylnitril, HCN, Acetonitril, Wasserdampf und Verunreinigungen umfasst, zu einer Abschrecksäule (102),
Kontaktieren des Reaktorabgases mit Abschreckflüssigkeit in der Abschrecksäule (102), um Folgendes bereitzustellen:
(a) einen Bodenstrom der Abschrecksäule, der Wasser und Verunreinigungen enthält, der durch eine Leitung (106) entfernt und zu Abfallbehandlung gesendet wird, und
(b) einen Strom aus gekühlten Reaktorabgasen;
Leiten der gekühlten Reaktorabgase zu einem Abschrecknachkühler (107),
Zuführen des gekühlten Abschreckabflusses aus dem Abschrecknachkühler (107) zu einem Absorber (110) und Zuführen von Waschwasser, das an der Oberseite des Absorbers eintritt, und Entfernen von Folgendem aus dem Absorber (110):
(a) nicht kondensierbaren Gasen, und
(b) einer wässrigen Lösung, die Wasser, Acrylnitril, Acetonitril und Verunreinigungen enthält, die als Bodenstrom entfernt wird;
Leiten des Bodenstroms zu einer Extraktivdestillationssäule (182) und Einführen von Lösungsmittelwasser in die Oberseite der Extraktivdestillationssäule (182), um Extraktivdestillation durchzuführen, und Entfernen von Folgendem aus der Extraktivdestillationssäule (182):
(a) Acrylnitril und HCN als Überkopfdampf, der zu weiterer Reinigung gesendet wird,
(b) einen Strom, der Acetonitril und Wasser enthält, und
(c) einen Bodenstrom, der Wasser, schwere organische Stoffe und andere Verunreinigungen enthält;
Leiten des Stroms, der Acetonitril und Wasser enthält, aus der Extraktivdestillationssäule (182) zu einem Stripper (190), dem Wärme zugeführt wird, und Entfernen von Folgendem aus dem Stripper (190):
(i) Acetonitril als Überkopfdampf, und
(ii) einen Flüssigkeitsstrom, der hauptsächlich Wasser enthält, aus der unteren Hälfte des Strippers (190);
Bereitstellen des Bodenstroms, der Wasser, schwere organische Stoffe und andere Verunreinigungen enthält, aus der Extraktivdestillationssäule (182) als einen Prozessstrom (196) an ein Verdampfersystem mit einer oder mehreren Verdampfungsstufen,
Abtrennen von Wasser von den schweren organischen Verunreinigungen in dem Verdampfersystem mit einer oder mehreren Verdampfungsstufen, um ein wässriges Kondensat und einen flüssigen Rückstand bereitzustellen, und
Kontaktieren des flüssigen Rückstands mit dem Reaktorausfluss in der Abschrecksäule (102), um einen Bodenstrom der Abschrecksäule bereitzustellen, der Ammoniumsulfat und Polymer umfasst; wobei die Menge an Ammoniumsulfat und die Menge an Polymer in dem Bodenstrom der Abschrecksäule durch eine Formel y = -M1x + C1 definiert sind, wobei y ein Gew.-% von Ammoniumsulfat ist, x ein Gew.-% Polymer ist, M1 4,6 ist und C1 45 oder weniger ist.

2. Verfahren nach Anspruch 1, wobei das wässrige Kondensat 0,1 Gewichtsprozent oder weniger schwere organische Verunreinigungen aufweist und der flüssige Rückstand 3 bis 10 Gew.-% schwere organische Verunreinigungen aufweist.

3. Verfahren nach Anspruch 1, wobei der Verfahrensstrom 0,5 bis 1,5 Gewichtsprozent schwere organische Verunreinigungen beinhaltet und wobei der Verfahrensstrom bevorzugt 0,75 bis 1,25 Gewichtsprozent schwere organische Verunreinigungen beinhaltet.

4. Verfahren nach Anspruch 1, wobei die schweren organischen Verunreinigungen polymere Materialien beinhalten, die in einem Ammoxidationsreaktionsverfahren produziert werden.

5. Verfahren nach Anspruch 1, wobei das Verdampfersystem 1 bis 6 Verdampfungsstufen beinhaltet, wobei das Verdampfersystem bevorzugt 2 bis 6 Verdampfungsstufen beinhaltet, wie wobei das Verdampfersystem 2 bis 5 Verdampfungsstufen beinhaltet, wobei das Verdampfersystem bevorzugter 2 bis 4 Verdampfungsstufen beinhaltet und wobei das Verdampfersystem am bevorzugtesten 2 bis 3 Verdampfungsstufen beinhaltet.

6. Verfahren nach Anspruch 4, wobei das Ammoxidationsreaktionsverfahren ein Acrylnitrilverfahren ist.

7. Verfahren nach Anspruch 1, wobei das wässrige Kondensat 0,075 Gewichtsprozent oder weniger schwere organische Verunreinigungen aufweist, wobei das wässrige Kondensat bevorzugt 0,05 Gewichtsprozent oder weniger schwere organische Verunreinigungen aufweist, und wobei das wässrige Kondensat am bevorzugtesten 0,025 Gewichtsprozent oder weniger schwere organische Verunreinigungen aufweist.

8. Verfahren nach Anspruch 1, wobei mindestens ein Teil des wässrigen Kondensats zu einer Abschrecksäule und/oder einem Stripper für leichte organische Stoffe befördert wird, und wobei bevorzugt mindestens ein Teil des wässrigen Kondensats zu einer ersten Stufe einer Abschrecksäule befördert wird.

9. Verfahren nach Anspruch 1, wobei das Verdampfersystem zum Bereitstellen eines Gesamtverdampfungsprozentsatzes von mehr als 55 % bis 85 % wirksam ist, wobei das Verdampfersystem bevorzugt zum Bereitstellen eines Gesamtverdampfungsprozentsatzes von mehr als 60 % bis 85 % wirksam ist, und wobei das Verdampfersystem am bevorzugtesten zum Bereitstellen eines Gesamtverdampfungsprozentsatzes von mehr als 73 % bis 75 % wirksam ist.

10. Verfahren nach Anspruch 1, wobei der flüssige Rückstand 4 bis 8 Gewichtsprozent schwere organische Verunreinigungen aufweist und wobei der flüssige Rückstand bevorzugt 5 bis 7 Gewichtsprozent schwere organische Verunreinigungen aufweist.

11. Verfahren nach Anspruch 1, wobei das Verdampfersystem mindestens einen Rohrbündelwärmetauscher beinhaltet und wobei bevorzugt eine Strömung durch eine Rohrseite des Wärmetauschers 1 bis 3 Meter/Sekunde beträgt.

12. Verfahren nach Anspruch 1, wobei jede Verdampfungsstufe eine Verdampfungsrate von 15 bis 25 % bereitstellt.

13. Verfahren nach Anspruch 1, wobei Schwefelsäure zu der Abschrecksäule befördert wird, um Bodenströme einer Abschrecksäule bereitzustellen, die 10 bis 25 Gew.-% Ammoniumsulfat und weniger als 5 Gew.-% Polymer aufweisen, und wobei bevorzugt Schwefelsäure zu der Abschrecksäule befördert wird, um Bodenströme einer Abschrecksäule bereitzustellen, die 15 bis 21 Gew.-% Ammoniumsulfat und weniger als 5 Gew.-% Polymer aufweisen.

14. Verfahren nach Anspruch 1, wobei der Bodenstrom der Abschrecksäule einen pH-Wert von 4,5 bis 6,0 aufweist, und wobei bevorzugt der pH-Wert mit Zufügen von Säure kontrolliert wird.

15. Verfahren nach Anspruch 1, wobei die Abfallbehandlung des Bodenstroms der Abschrecksäule Entfernung von Wasser involviert, um die Konzentration von Ammoniumsulfat darin zu erhöhen.

## Revendications

1. Procédé de fourniture d'un résidu liquide à un courant de procédé d'ammoxydation, le procédé comprenant :
le fait de faire passer un gaz effluent de réacteur comprenant de l'acrylonitrile, du HCN, de l'acétonitrile, de la vapeur d'eau et des impuretés dans une colonne de trempe (102),
la mise en contact du gaz effluent de réacteur avec un liquide de trempe dans la colonne de trempe (102), pour fournir :
(a) un courant de fond de colonne de trempe contenant de l'eau et des impuretés qui est évacué par un conduit (106) et envoyé à un traitement des déchets, et
(b) un courant de gaz effluents de réacteur refroidis ;
le fait de faire passer les gaz effluents de réacteur refroidis dans un post-refroidisseur de trempe (107),
l'alimentation d'un absorbeur (110) en l'effluent de trempe refroidi provenant du post-refroidisseur de trempe (107) et l'alimentation en eau de lavage qui entre par le sommet de l'absorbeur, et l'élimination de l'absorbeur (110) :
(a) des gaz non condensables, et
(b) d'une solution aqueuse contenant de l'eau, de l'acrylonitrile, de l'acétonitrile et des impuretés qui est éliminée en tant que courant de fond ;
le fait de faire passer le courant de fond dans une colonne de distillation extractive (182), et l'introduction de l'eau de solvant au sommet de la colonne de distillation extractive (182) pour effectuer une distillation extractive, et l'élimination de la colonne de distillation extractive (182) :
(a) de l'acrylonitrile et du HCN sous forme de vapeur de tête qui est envoyée à une purification supplémentaire,
(b) d'un courant contenant de l'acétonitrile et de l'eau, et
(c) d'un courant de fond contenant de l'eau, des matières organiques lourdes et d'autres impuretés ;
le fait de faire passer le courant contenant de l'acétonitrile et de l'eau provenant de la colonne de distillation extractive (182) dans un séparateur (190), auquel de la chaleur est ajoutée, et l'élimination du séparateur (190) :
(i) de l'acétonitrile sous forme de vapeur de tête, et
(ii) d'un courant liquide contenant principalement de l'eau provenant de la moitié inférieure du séparateur (190) ;
la fourniture du courant de fond contenant de l'eau, des matières organiques lourdes et d'autres impuretés de la colonne de distillation extractive (182) en tant que courant de traitement (196) à un système évaporateur ayant un ou plusieurs étages d'évaporation,
la séparation de l'eau des impuretés organiques lourdes dans le système évaporateur ayant un ou plusieurs étages d'évaporation pour fournir un condensat aqueux et un résidu liquide, et
la mise en contact du résidu liquide avec l'effluent de réacteur dans la colonne de trempe (102) pour fournir un courant de fond de colonne de trempe comprenant du sulfate d'ammonium et un polymère ;
dans lequel la quantité de sulfate d'ammonium et la quantité de polymère dans le courant de fond de colonne de trempe sont définies par une formule y = -M1x + C1 où y est un % en poids de sulfate d'ammonium, x est un % en poids de polymère, M1 vaut 4,6 et C1 est inférieur ou égal à 45.

2. Procédé selon la revendication 1, dans lequel le condensat aqueux contient 0,1 pourcent en poids ou moins d'impuretés organiques lourdes et le résidu liquide a 3 à 10 % en poids d'impuretés organiques lourdes.

3. Procédé selon la revendication 1, dans lequel le courant de procédé comporte 0,5 à 1,5 pourcent en poids d'impuretés organiques lourdes, et de préférence dans lequel le courant de procédé comporte 0,75 à 1,25 pourcent en poids d'impuretés organiques lourdes.

4. Procédé selon la revendication 1, dans lequel les impuretés organiques lourdes comportent des matériaux polymères produits dans un procédé de réaction d'ammoxydation.

5. Procédé selon la revendication 1, dans lequel le système évaporateur comporte 1 à 6 étages d'évaporation, de préférence dans lequel le système évaporateur comporte 2 à 6 étages d'évaporation, tel que dans lequel le système évaporateur comporte 2 à 5 étages d'évaporation, plus préférablement dans lequel le système évaporateur comporte 2 à 4 étages d'évaporation, et idéalement dans lequel le système évaporateur comporte 2 à 3 étages d'évaporation.

6. Procédé selon la revendication 4, dans lequel le procédé de réaction d'ammoxydation est un procédé à l'acrylonitrile.

7. Procédé selon la revendication 1, dans lequel le condensat aqueux contient 0,075 pourcent en poids ou moins d'impuretés organiques lourdes, de préférence dans lequel le condensat aqueux contient 0,05 pourcent en poids ou moins d'impuretés organiques lourdes, et idéalement dans lequel le condensat aqueux contient 0,025 pourcent en poids ou moins d'impuretés organiques lourdes.

8. Procédé selon la revendication 1, dans lequel au moins une partie du condensat aqueux est acheminée vers une colonne de trempe et/ou un séparateur de matières organiques légères, et de préférence dans lequel au moins une partie du condensat aqueux est acheminée vers un premier étage d'une colonne de trempe.

9. Procédé selon la revendication 1, dans lequel le système évaporateur est efficace pour assurer un pourcentage d'évaporation global supérieur à 55 % à 85 %, de préférence dans lequel le système évaporateur est efficace pour assurer un pourcentage d'évaporation global supérieur à 60 % à 85 %, et idéalement dans lequel le système évaporateur est efficace pour assurer un pourcentage d'évaporation global supérieur à 73 % à 75 %.

10. Procédé selon la revendication 1, dans lequel le résidu liquide contient 4 à 8 pourcent en poids d'impuretés organiques lourdes, et de préférence dans lequel le résidu liquide contient 5 à 7 pourcent en poids d'impuretés organiques lourdes.

11. Procédé selon la revendication 1, dans lequel le système évaporateur comporte au moins un échangeur de chaleur à calandre et tube, et de préférence dans lequel un débit à travers un côté tube de l'échangeur de chaleur est de 1 à 3 mètres/seconde.

12. Procédé selon la revendication 1, dans lequel chaque étage d'évaporation assure un taux d'évaporation de 15 à 25 %.

13. Procédé de la revendication 1, dans lequel l'acide sulfurique est acheminé vers la colonne de trempe pour fournir des courants de fond de colonne de trempe ayant 10 à 25 % en poids de sulfate d'ammonium et moins de 5 % en poids de polymère, et de préférence dans lequel l'acide sulfurique est acheminé vers la colonne de trempe pour fournir des courants de fond de colonne de trempe ayant 15 à 21 % en poids de sulfate d'ammonium et moins de 5 % en poids de polymère.

14. Procédé selon la revendication 1, dans lequel le courant de fond de colonne de trempe a un pH de 4,5 à 6, 0, et de préférence dans lequel le pH est contrôlé par l'ajout d'un acide.

15. Procédé selon la revendication 1, dans lequel le traitement des déchets du courant de fond de colonne de trempe implique l'élimination d'eau pour augmenter la concentration de sulfate d'ammonium dans celui-ci.
